# EUROPEAN PATENT APPLICATION

(11) **EP 3 483 596 A1**
(43) Date of publication of application: **15.05.2019**
(21) Application number: 17823943.0
(22) Date of filing: 12.06.2017
(51) Int. Cl.: G01N 27/12

(54) **GAS SENSOR**

(30) Priority: 08.07.2016 JP 2016135695
(71) Applicant: NGK Spark Plug Co., Ltd., Nagoya-shi, Aichi 467-8525 (JP)
(72) Inventor: FURUSAKI, Keizo, Nagoya-shi Aichi 467-8525 (JP); UEKI, Masatoshi, Nagoya-shi Aichi 467-8525 (JP); NISHIO, Kenji, Nagoya-shi Aichi 467-8525 (JP); INOUE, Tsuyoshi, Nagoya-shi Aichi 467-8525 (JP)
(74) Representative: J A Kemp
(86) International application number: PCT/JP2017/021660
(87) International publication number: WO 2018/008336

(57) **Abstract**

A gas sensor which can improve the accuracy in detecting a particular gas component and can realize a reduction in size and electric power savings is provided.

A gas sensor 1A includes an adjustment unit 10 having a concentration adjuster 14 which changes the concentration of a particular gas component contained in gas G to be measured which is introduced into a first chamber C1; a sensor unit 20 having a second chamber C2 for receiving the a gas to be measured which has passed through the adjustment unit, and a detector 24a whose electrical characteristic changes with the concentration of the particular gas component; a single heater 24b for heating the concentration adjuster and the detector; and a gas flow tube 40 having the form of a pipe, at least partially running externally of the adjustment unit and of the sensor unit, and adapted to establish communication between the first chamber and the second chamber. The adjustment unit, the sensor unit, and the heater are united together in such a manner as to establish thermal coupling between the adjustment unit and the heater and thermal coupling between the sensor unit and the heater.

## Description

### TECHNICAL FIELD

The present invention relates to a gas sensor for detecting the concentration of a particular gas component contained in a gas to be measured.

### BACKGROUND ART

Conventionally, there is known a gas sensor for detecting the concentration of a particular gas component contained in gas to be measure (Patent Document 1).

According to this gas sensor, the air (gas to be measured) is supplied in a fixed amount into a chamber; the supplied gas to be measured is pretreated within the chamber for removing flammable gases such as CO through burning; and the pretreated gas to be measured is brought into contact with a sensor element for detecting the concentration of NOₓ.

### PRIOR ART DOCUMENT

### PATENT DOCUMENT

Patent Document 1: Japanese Patent Application Laid-Open (*kokai*) No. H10-300702

### SUMMARY OF THE INVENTION

### PROBLEM TO BE SOLVED BY THE INVENTION

According to Patent Document 1, separate heaters are provided for a pretreatment section adapted to remove gases which affect the detection of the concentration of a particular gas component and for a sensor element adapted to detect the concentration of the particular gas component. The separate heaters heat the pretreatment section and the sensor element, respectively, so as to activate the pretreating function and the sensing function. However, the provision of separate heaters for activating the pretreating function which changes the concentration of a particular gas component, and for activating the sensing function, involves the following problems: the size of the gas sensor increases as a result of an increase in the number of heaters, and the power consumption of the heaters increases as a result of an increase in waste heat from the heaters. Meanwhile, in order to improve accuracy in detecting a particular gas component, at least a gas detector must be heated to an operating temperature or higher; therefore, provision of no heater is not realistic.

In view of the above, an object of the present invention is to provide a gas sensor which can improve the accuracy in detecting a particular gas component and can realize a reduction in size and electric power savings.

### MEANS FOR SOLVING THE PROBLEM

In order to solve the above problem, a gas sensor of the present invention comprises an adjustment unit having a first chamber into which a gas to be measured is introduced, and a concentration adjuster which changes the concentration of a particular gas component contained in the gas to be measured which is introduced into the first chamber; a sensor unit having a second chamber for receiving the gas to be measured which has passed through the adjustment unit, and a detector whose electrical characteristic changes with the concentration of the particular gas component; a single heater for heating the concentration adjuster and the detector; and a gas flow tube having the form of a pipe, at least partially running externally of the adjustment unit and of the sensor unit, and adapted to establish communication between the first chamber and the second chamber. The adjustment unit, the sensor unit, and the heater are united together in such a manner as to establish thermal coupling between the adjustment unit and the heater and thermal coupling between the sensor unit and the heater.

According to this gas sensor, since a single heater heats the adjustment unit and the sensor unit in contrast to the case of provision of separate heaters for heating the adjustment unit and the sensor unit, the gas sensor can be reduced in size and can save electric power. Also, by means of the heater heating the detector to an operating temperature, the particular gas component can be stably detected, and the accuracy in detecting the particular gas component can be improved.

Further, since the gas to be measured whose concentration of the particular gas component has been adjusted in the adjustment unit is introduced into the sensor unit through the gas flow tube free from influence of the heater, for example, the gas to be measured in transmission from the adjustment unit to the sensor unit is free from a further change of the concentration, which could otherwise result from the gas to be measured being unexpectedly heated by the heater in the course of transmission from the adjustment unit to the sensor unit. Thus, the gas to be measured which has been treated in the adjustment unit can be introduced intact into the sensor unit, whereby the accuracy in detecting the particular gas component can be further improved.

The gas sensor according to claim 1 may be configured such that the single heater has a first surface and a second surface opposite to each other; the concentration adjuster is disposed on one side of the heater where the first surface is present; and the detector is disposed on the other side of the heater where the second surface is present.

According to this gas sensor, since the concentration adjuster and the detector are disposed respectively on opposite sides of the heater, heat of the heater can be transmitted to the concentration adjuster and to the detector with no waste, and electric power savings can be implemented. The concentration adjuster may be disposed on the first surface side of the heater such that the concentration adjuster is disposed directly on the first surface of the heater or indirectly with another member intervening therebetween. Also, the detector may be disposed on the second surface side of the heater such that the detector is disposed directly on the second surface of the heater or indirectly with another member intervening therebetween.

The gas sensor according to claim 2 may be configured such that the detector is disposed on the second surface of the single heater, and the concentration adjuster is disposed on the side of the heater where the first surface is present with a heat insulating layer intervening between the concentration adjuster and the heater.

According to this gas sensor, in spite of use of a single heater, the detector can be appropriately maintained at an operating temperature, whereby the accuracy in detecting the particular gas component can be improved.

The gas sensor according to claim 1 may be configured as follows: a common member is used to form a portion of a constituent member for defining the first chamber of the adjustment unit and a portion of a constituent member for defining the second chamber of the sensor unit, and the concentration adjuster is disposed on one side of the common member where the first chamber is present, while the detector is disposed on the other side of the common member where the second chamber is present, and the single heater intervenes between the concentration adjuster and the common member or between the detector and the common member.

According to this gas sensor, by virtue of employment of the common member, the number of components of the gas sensor can be reduced, and the size of the gas sensor can be further reduced.

The gas sensor according to claim 4 may be configured such that the single heater intervenes between the detector and the common member and such that a heat insulating layer is disposed between the heater and the common member.

According to this gas sensor, since the heater is disposed on the detector side of the common member, the heater can promptly heat the detector to an operating temperature, and the accuracy in detecting the particular gas component can be further improved.

### EFFECT OF THE INVENTION

The present invention provides a gas sensor which can improve the accuracy in detecting a particular gas component and can realize a reduction in size and electric power savings.

### BRIEF DESCRIPTION OF THE DRAWINGS

[FIG. 1] Exploded perspective view of a gas sensor according to a first embodiment of the present invention.
[FIG. 2] Sectional view taken along line A-A of FIG. 1.
[FIG. 3] Exploded perspective view of a gas sensor according to a second embodiment of the present invention.
[FIG. 4] Sectional view taken along line B-B of FIG. 3.

### MODES FOR CARRYING OUT THE INVENTION

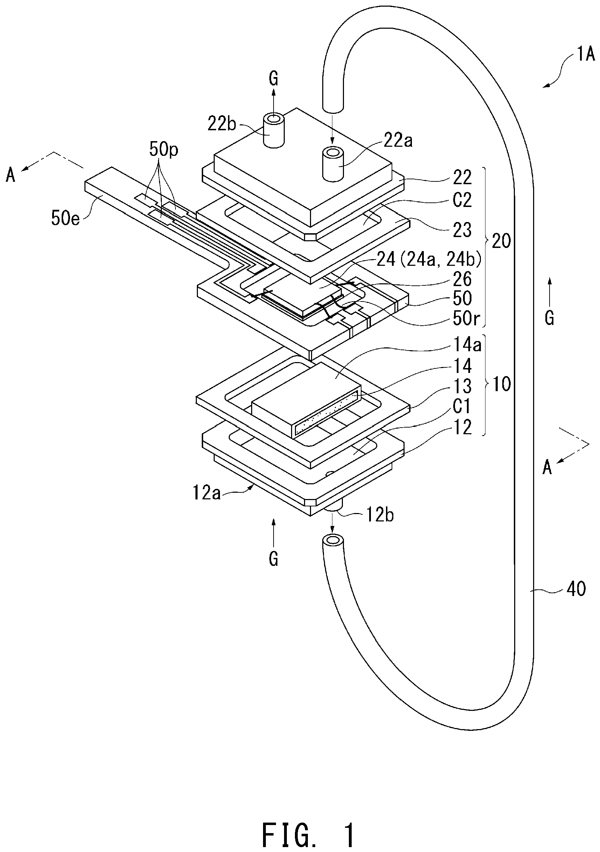

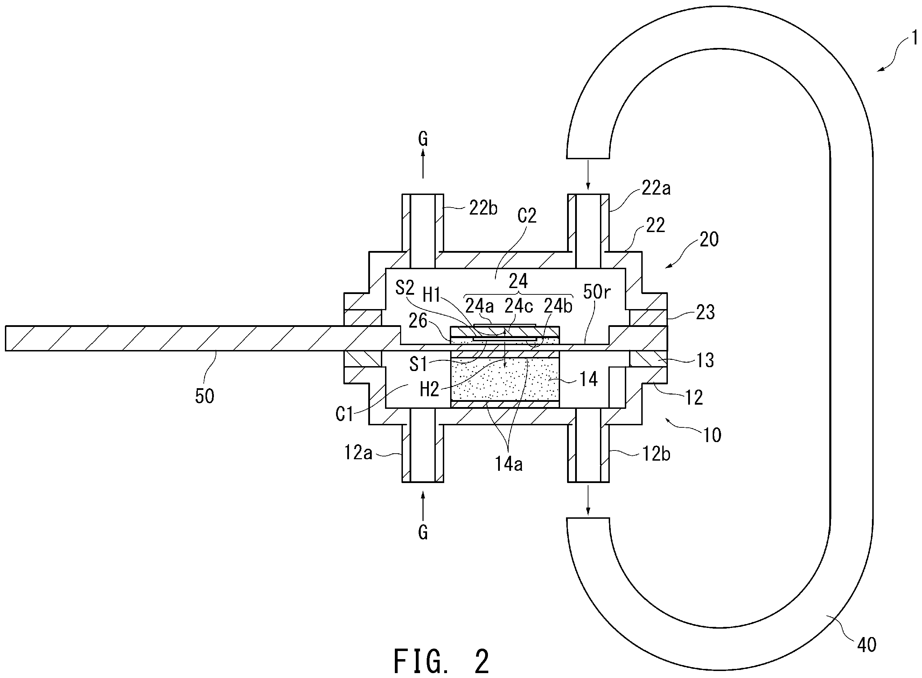

The present invention will next be described in detail with reference to the drawings. FIG. 1 is an exploded perspective view of a gas sensor 1A according to a first embodiment of the present invention. FIG. 2 is a sectional view taken along line A-A of FIG. 1.

In FIG. 1, the gas sensor 1A includes an adjustment unit 10, a sensor unit 20, a rubber tube (gas flow tube) 40 in the form of a pipe, and a plate-like ceramic wiring board 50. The gas sensor 1A as a whole has the form of a box.

The adjustment unit 10 has a metal case 12 having the form of an approximately rectangular box, having a flange, and opening upward (upward in FIG. 1), a seal material (packing) 13 having the form of a rectangular frame and in contact with the flange of the case 12, a concentration adjuster 14 accommodated within the case 12, and the above-mentioned ceramic wiring board 50. As a result of the flange of the case 12 and an outer peripheral portion of the lower surface of the ceramic wiring board 50 coming into contact with the frame of the seal material 13, the ceramic wiring board 50 closes the opening of the case 12, whereby the internal space of the case 12 forms a first chamber C1.

The case 12 has an inlet 12a and an outlet 12b each having the form of a pipe, protruding from its lower surface at positions located away from each other, serving as connections for piping, and communicating with the first chamber C1.

A porous, gas permeable concentration adjuster 14 is disposed within the first chamber C1 between the inlet 12a and the outlet 12b. A seal material 14a is provided on the surface of the concentration adjuster 14 for sealing a gap between the concentration adjuster 14 and the walls of the first chamber C1.

A gas G to be measured is introduced into the first chamber C1 from the inlet 12a, comes into contact with the concentration adjuster 14 to thereby be adjusted in the concentration of a particular gas component, and is then discharged from the outlet 12b to the outside of the adjustment unit 10. The concentration adjuster 14 is a structure for removing miscellaneous gases other than the particular gas component to thereby adjust the concentration of the particular gas component contained in the gas to be measured.

The sensor unit 20 has a metal case 22 having the same shape as that of the case 12 and opening downward, a seal material (packing) 23 having the form of a rectangular frame and bonded to the flange of the case 22 through an adhesive (not shown), a sensor element 24 accommodated within the case 22, a heat insulating sheet (heat insulating layer) 26 formed of an unwoven sheet of inorganic fiber, and the above-mentioned ceramic wiring board 50. As a result of the flange of the case 22 and an outer peripheral portion of the upper surface of the ceramic wiring board 50 being fixed to the frame of the seal material 23 through an adhesive (not shown), the ceramic wiring board 50 closes the opening of the case 22, whereby the internal space of the case 22 forms a second chamber C2.

The sensor element 24 has the form of an approximately rectangular plate and has, as shown in FIG. 2, a monolithic structure in which a detector 24a is laminated on the upper surface (surface facing upward in FIG. 1) of a base 24c, while a heater 24b is laminated on the lower surface of the base 24c.

The sensor element 24 is disposed on the ceramic wiring board 50 such that the heat insulating sheet 26 is disposed in a recess 50r formed in a central region of the upper surface of the ceramic wiring board 50 and such that the heater 24b is in contact with the upper surface of the heat insulating sheet 26.

The case 22 has an inlet 22a and an outlet 22b each having the form of a pipe, protruding from its upper surface at positions located away from each other, serving as connections for piping, and communicating with the second chamber C2.

The sensor element 24 is disposed in the recess 50r within the second chamber C2 between the inlet 22a and the outlet 22b. The inlet 22a is connected to the outlet 12b through the rubber tube 40. The gas G to be measured whose concentration of the particular gas component has been adjusted by passing through the adjustment unit 10 flows through the rubber tube 40, is introduced into the second chamber C2 from the inlet 22a, comes into contact with the detector 24a to thereby be measured for the concentration of the particular gas component, and is then discharged from the outlet 22b to the outside of the sensor unit 20.

The electrical characteristic of the detector 24a changes with the concentration of the particular gas component, and the concentration of the particular gas component is detected through detection of its changed electric signal. The heater 24b generates heat through energization and heats the detector 24a to an operating temperature. Output terminals of the detector 24a and energization terminals of the heater 24b are electrically connected to the ceramic wiring board 50 by wire bonding, for example.

The base 24c can be formed of an electrically insulative ceramic substrate, for example. The detector 24a can be formed by use of a metal oxide semiconductor, for example. The heater 24b can be implemented by, for example, a circuit formed on the surface of the base 24c and serving as a heat-generating resistor. The detector 24a may employ a publicly known structure in which electrodes are provided on a solid electrolyte member, for example.

An end portion 50e (located at the left of FIG. 1) of the ceramic board 50 is narrower than the cases 12 and 22, extends outward (leftward in FIG. 1), and has a plurality of electrode pads 50p formed on its opposite surfaces and electrically connected to the detector 24a and the heater 24b through the above-mentioned wire bonding and wiring (lead conductors) formed on the surface of the ceramic wiring board 50. The electric signal output from the detector 24 is output to an external device through the electrode pads 50p of the ceramic wiring board 50, and electricity supplied from outside through the electrode pads 50p energizes the heater 24b for generating heat.

As shown in FIG. 2, the sensor unit 20 and the heater 24b are thermally coupled as indicated by arrow H1 as a result of stacking of (establishment of contact between) the heater 24b and the detector 24a within the sensor unit 20 through the base 24c.

Similarly, the adjustment unit 10 and the heater 24b are thermally coupled as indicated by arrow H2 as a result of stacking of (establishment of contact between) the heater 24b and the concentration adjuster 14 within the adjustment unit 10 through the heat insulating sheet 26 and a portion of the printed board 50 where the recess 50r is formed.

The expression "the sensor unit 20 and the heater 24b are thermally coupled" means that a certain member which partially constitutes the sensor unit 20, and the heater 24b are coupled together with no air intervening therebetween (with no gap intervening therebetween). This encompasses, for example, the case where a heater separated from the detector 24 is thermally coupled with the case 22, which partially constitutes the sensor unit 20, and heats the space within the second chamber C2 through the case 22.

The same also applies to the expression "the adjustment unit 10 and the heater 24b are thermally coupled."

As mentioned above, since the single heater 24b heats the adjustment unit 10 and the sensor unit 20 in contrast to the case of provision of separate heaters for heating the adjustment unit 10 and the sensor unit 20, the gas sensor 1A can be reduced in size and can save electric power. Also, by means of the heater 24b heating the detector 24a to an operating temperature, the particular gas component can be stably detected, and the accuracy in detecting the particular gas component can be improved.

Further, since the gas G to be measured whose concentration of the particular gas component has been adjusted in the adjustment unit 10 is introduced into the sensor unit 20 through the rubber tube 40 free from influence of the heater 24b, for example, the gas G to be measured in transmission from the adjustment unit 10 to the sensor unit 20 is free from a further change of the concentration, which could otherwise result from the gas G to be measured being unexpectedly heated by the heater in the course of transmission from the adjustment unit 10 to the sensor unit 20. Thus, the gas G to be measured which has been treated in the adjustment unit 10 can be introduced intact into the sensor unit 20, whereby the accuracy in detecting the particular gas component can be further improved.

As shown in FIG. 2, in the first embodiment, the heater 24b has the form of a plate and has a lower surface (first surface) S1 and an upper surface (second surface) S2 opposite to each other; the concentration adjuster 14 is disposed on the lower surface S1 side; and the detector 24a is disposed on the upper surface S2 side.

Thus, since the concentration adjuster 14 and the detector 24a are disposed respectively on opposite sides of the heater 24b, heat of the heater 24b can be transmitted to the concentration adjuster 14 and to the detector 24a with no waste, and electric power can be saved further.

Also, in the first embodiment, the concentration adjuster 14 is disposed on the lower surface S1 side of the heater 24b with the heat insulating sheet 26 intervening therebetween.

By virtue of this, in spite of use of the single heater 24b, the detector 24a can be appropriately maintained at an operating temperature.

Also, in the first embodiment, the ceramic wiring board 50 is used as a common member to form a portion of a constituent member for defining the first chamber C1 of the adjustment unit 10 and a portion of a constituent member for defining the second chamber C2 of the sensor unit 20.

The concentration adjuster 14 is disposed on a side toward the first chamber C1 (on a lower surface side) of the ceramic wiring board 50, while the detector 24a is disposed on a side toward the second chamber C2 (on an upper surface side) of the ceramic wiring board 50. Further, the heater 24b intervenes between the detector 24a and the ceramic wiring board 50.

Thus, by virtue of the ceramic wiring board 50 serving as a common member, the number of components of the gas sensor 1A can be reduced, and the size of the gas sensor 1A can be further reduced.

Also, in the first embodiment, the heat insulating sheet 26 is disposed between the heater 24b and the ceramic wiring board 50.

Thus, since the heater 24b is disposed on the detector 24a side of the ceramic wiring board 50, the heater 24b can promptly heat the detector 24a to an operating temperature, and the accuracy in detecting the particular gas component can be further improved.

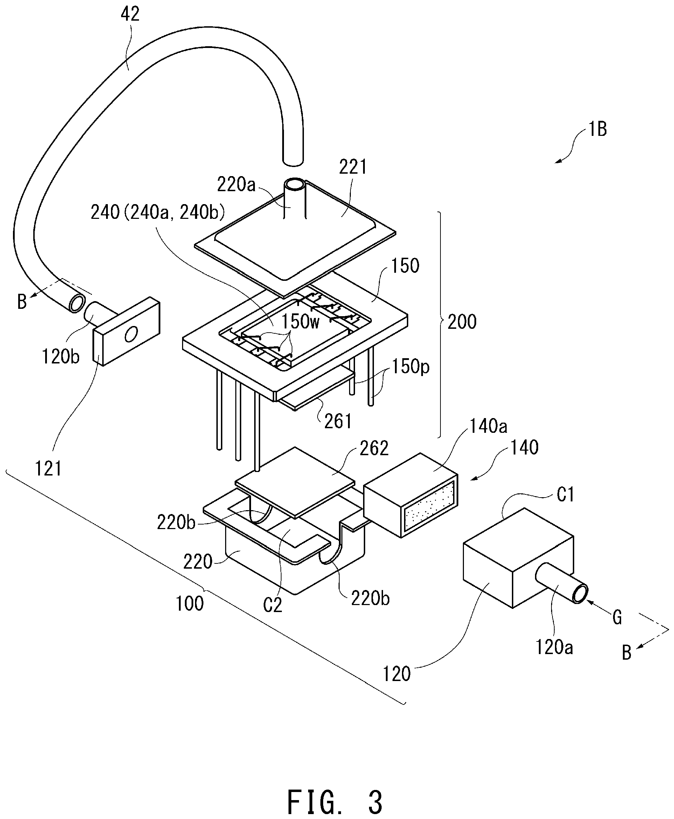

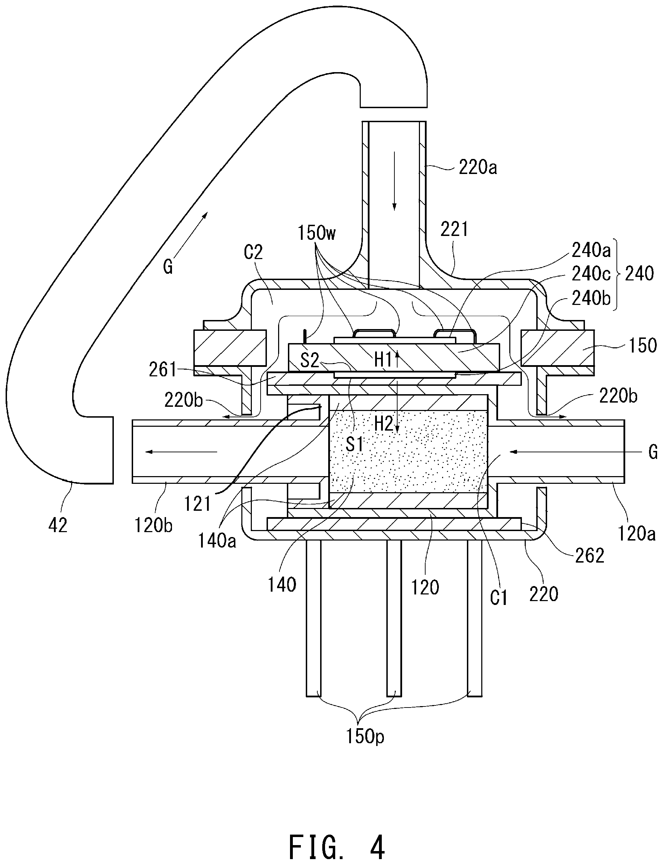

Next, with reference to FIGS. 3 and 4, a gas sensor 1B according to a second embodiment of the present invention will be described. FIG. 3 is an exploded perspective view of the gas sensor 1B, and FIG. 4 is a sectional view taken along line B-B of FIG. 3.

In FIG. 3, the gas sensor 1B includes an adjustment unit 100, a sensor unit 200, and a rubber tube (gas flow tube) 42. The gas sensor 1B as a whole has the form of a box.

The adjustment unit 100 has a first case 120 made of metal, having the form of an approximately rectangular box, and opening sideward (leftward in FIG. 3), a lid-like second case 121 made of metal and closing the opening of the first case 120, and a concentration adjuster 140 accommodated within the first case 120. The internal space of the first case 120 closed by the second case 121 forms the first chamber C1.

The first case 120 has an inlet 120a having the form of a pipe, protruding from its side surface (right side surface in FIG. 3) opposite the opening, and serving as a connection for piping. Similarly, the second case 121 has an outlet 120b having the form of a pipe, protruding from its side surface opposite the first case 120, and serving as a connection for piping. The inlet 120a and the outlet 120b communicate with the first chamber C1.

The concentration adjuster 140 is disposed within the first chamber C1 between the inlet 120a and the outlet 120b. A seal material 140a is provided on the surface of the concentration adjuster 140 for sealing a gap between the concentration adjuster 140 and the walls of the first chamber C1. The concentration adjuster 140 is a gas permeable structure for removing miscellaneous gases other than the particular gas component to thereby adjust the concentration of the particular gas component contained in the gas to be measured.

The gas G to be measured is introduced into the first chamber C1 from the inlet 120a, comes into contact with the concentration adjuster 140 to thereby be adjusted in the concentration of the particular gas component, and is then discharged from the outlet 120b to the outside of the adjustment unit 100.

As shown in FIG. 4, in the second embodiment, the first case 120 is entirely filled with the concentration adjuster 140, and the first chamber C1 is the internal space of the inlet 120a of the first case 120.

The sensor unit 200 has a first case 220 made of metal, having the form of an approximately rectangular box, having a flange, and opening upward, a second case 221 made of metal, having the form of a lid, having a flange, and opening downward, a ceramic wiring board 150 having the form of a rectangular frame, a sensor element 240 fixed within the frame of the ceramic wiring board 150 by wire bonding 150w, and two heat insulating sheets (heat insulating layers) 261 and 262.

The first case 220 has U-shaped cuts (outlets) 220b formed in its opposite sides, respectively, and extending downward from the flange. The adjustment unit 100 is disposed within the first case 220 through the heat insulating sheet 262 such that the inlet 120a and the outlet 120b protrude outward from the first case 220 through the two cuts 220b.

Further, the ceramic wiring board 150 is disposed on the upper surface of the adjustment unit 100 (first case 120) through the heat insulating sheet 261, and the second case 221 is disposed on the ceramic wiring board 150. The flanges of the first case 220 and the second case 221 are bonded to the frame of the ceramic wiring board 150 through an adhesive (not shown), whereby the second case 221 closes the opening of the first case 220; as a result, internal spaces of the first case 220 and the second case 221 located externally of the adjustment unit 100 form the second chamber C2.

The sensor element 240 is fixedly suspended, by the wire bonding 150w, inside the frame of the ceramic wiring board 150 in the second chamber C2 and protrudes downward of the ceramic wiring board 150 to come into contact with the heat insulating sheet 261.

The sensor element 240 has the form of an approximately rectangular plate and has a structure similar to that of the sensor element 24 of the first embodiment. Specifically, as shown in FIG. 4, the sensor element 240 has a monolithic structure in which a detector 240a is disposed on the upper surface (surface facing upward in FIG. 3) of a base 240c, while a heater 240b is disposed on the lower surface of the base 240c; i.e., the detector 240a and the heater 240b are disposed on the upper and lower sides of the base 240c. Since the detector 240a and the heater 240b have structures similar to those of the detector 24a and the heater 24b in the first embodiment, repeated description thereof is omitted.

The heater 240b is in contact with the heat insulating sheet 261.

The second case 221 has an inlet 220a having the form of a pipe, protruding from its upper surface, serving as a connection for piping, and communicating with the second chamber C2. The inlet 220a is connected to the outlet 120b through the rubber tube 42.

The gas G to be measured whose concentration of the particular gas component has been adjusted by passing through the adjustment unit 100 flows through the rubber tube 42, is introduced into the second chamber C2 from the inlet 220a, comes into contact with the detector 240a to thereby be measured for the concentration of the particular gas component, passes through the frame of the printed board 150, and is then discharged from the cuts 220b to the outside of the sensor unit 200.

Notably, as shown in FIG. 4, the two cuts 220b are greater in size than the outside diameters of the inlet 120a and the outlet 120b, whereby gaps between the cuts 220b and the inlet 120a and the outlet 120b serve as outlets.

Output terminals of the detector 240a and energization terminals of the heater 240b are electrically connected to the ceramic wiring board 150 by the wire bonding 150w, and a plurality of pin terminals 150p electrically connected to the wire bonding 150w protrude downward from the ceramic wiring board 150. An electric signal output from the detector 240a is output to an external device through the terminals 150p of the ceramic wiring board 150, and electricity supplied from outside through the terminals 150p energizes the heater 240b for generating heat.

As shown in FIG. 4, the sensor unit 200 and the heater 240b are thermally coupled as indicated by arrow H1 as a result of stacking of (establishment of contact between) the heater 240b and the detector 240a within the sensor unit 200 through the base 240c.

Similarly, the adjustment unit 100 and the heater 240b are thermally coupled as indicated by arrow H2 as a result of stacking of (establishment of contact between) the heater 240b and the concentration adjuster 140 within the adjustment unit 100 through the heat insulating sheet 261 and the first case 120.

As shown in FIG. 4, in the second embodiment also, the heater 240b has the form of a plate and has the lower surface (first surface) S1 and the upper surface (second surface) S2 opposite to each other; the concentration adjuster 140 is disposed on the lower surface S1 side; and the detector 240a is disposed on the upper surface S2 side.

In the second embodiment also, the concentration adjuster 140 is disposed on the lower surface S1 side of the heater 240b with the heat insulating sheet 261 intervening therebetween.

In the second embodiment also, the first case 120 (and the second case 121) is used as a common member to form a portion of a constituent member for defining the first chamber C1 of the adjustment unit 100 and a portion of a constituent member for defining the second chamber C2 of the sensor unit 200.

The concentration adjuster 140 is disposed on a side toward the first chamber C1 (on a lower surface side) of the first case 120, while the detector 240a is disposed on a side toward the second chamber C2 (on an upper surface side) of the first case 120. Further, the heater 240b intervenes between the detector 240a and the first case 120.

In the second embodiment also, the heat insulating sheet 261 is disposed between the heater 240b and the first case 120.

The present invention is not limited to the above embodiments, but extends into various modifications and equivalents encompassed by the ideas and scope of the invention.

The gas sensor, and the adjustment unit and the sensor unit which partially constitute the gas sensor, are not limited in shape, etc., to the above embodiments. Also, the concentration adjuster and the detector are not limited in type, etc. The gas flow tube is not limited to a rubber tube, but may be a metal pipe, a resin pipe, or a tube formed by connecting a metal pipe and a rubber tube, for example.

### DESCRIPTION OF REFERENCE NUMERALS

1A, 1B: gas sensor
10, 100: adjustment unit
14, 140: concentration adjuster
20, 200: sensor unit
24, 240: sensor element
24a, 240a: detector
24b, 240b: heater
26, 261: heat insulating sheet (heat insulating layer)
40, 42: gas flow tube
50, 120, 121: common member (printed board, first case, second case)
C1: first chamber
C2: second chamber
G: gas to be measured
S1: first surface
S2: second surface

## Claims

1. A gas sensor comprising:
an adjustment unit having a first chamber into which a gas to be measured is introduced, and a concentration adjuster which changes the concentration of a particular gas component contained in the gas to be measured which is introduced into the first chamber;
a sensor unit having a second chamber for receiving the gas to be measured which has passed through the adjustment unit, and a detector whose electrical characteristic changes with the concentration of the particular gas component;
a single heater for heating the concentration adjuster and the detector; and
a gas flow tube having the form of a pipe, at least partially running externally of the adjustment unit and of the sensor unit, and adapted to establish communication between the first chamber and the second chamber;
wherein the adjustment unit, the sensor unit, and the heater are united together in such a manner as to establish thermal coupling between the adjustment unit and the heater and thermal coupling between the sensor unit and the heater.

2. A gas sensor according to claim 1, wherein the single heater has a first surface and a second surface opposite to each other; the concentration adjuster is disposed on one side of the heater where the first surface is present; and the detector is disposed on the other side of the heater where the second surface is present.

3. A gas sensor according to claim 2, wherein the detector is disposed on the second surface of the single heater, and the concentration adjuster is disposed on the side of the heater where the first surface is present with a heat insulating layer intervening between the concentration adjuster and the heater.

4. A gas sensor according to claim 1, wherein
a common member is used to form a portion of a constituent member for defining the first chamber of the adjustment unit and a portion of a constituent member for defining the second chamber of the sensor unit, and the concentration adjuster is disposed on one side of the common member where the first chamber is present, while the detector is disposed on the other side of the common member where the second chamber is present, and
the single heater intervenes between the concentration adjuster and the common member or between the detector and the common member.

5. A gas sensor according to claim 4, wherein the single heater intervenes between the detector and the common member, and a heat insulating layer is disposed between the heater and the common member.
